# EUROPEAN PATENT SPECIFICATION

(11) **EP 1 444 667 B1**
(45) Date of publication and mention of the grant of the patent: **08.03.2006**
(21) Application number: 02767029.8
(22) Date of filing: 17.10.2002
(51) Int. Cl.: G08B 15/00

(54) **FACE IMAGING SYSTEM FOR RECORDAL AND AUTOMATED IDENTITY CONFIRMATION**
GESICHTSBILDERZEUGUNGSSYSTEM ZUR BILDAUFNAHME UND AUTOMATISIERTE BESTÄTIGUNG DER IDENTITÄT
SYSTEME D'IMAGERIE DESTINE A L'ENREGISTREMENT D'IMAGES DE VISAGES ET A LA CONFIRMATION AUTOMATIQUE DE L'IDENTITE D'UNE IMAGE DE VISAGE

(30) Priority: 17.10.2001 CA 2359269
(43) Date of publication of application: 11.08.2004
(73) Proprietor: Biodentity Systems Corporation, Kanata, Ontario K2M 1P6 (CA)
(72) Inventor: VAN BEEK, Gary, A., North Gower, Ontario K0A 2T0 (CA); ADLER, Andrew, James, Ottawa, Ontario K1S 0T5 (CA); CORDEA, Marius, Daniel, Ottawa, Ontario K1N 1B3 (CA); MOICA, Simion, Adrian, Ottawa, Ontario K2C 0X5 (CA); ROSS, William, R., Merrickville, Ontario K0G 1N0 (CA); SHAW, Joel, F., Stittsville, Ontario K2S 1B4 (CA)
(74) Representative: Hill, Richard
(86) International application number: PCT/CA2002/001566
(87) International publication number: WO 2003/034361

(56) References cited:
- US-A- 6 122 445
- US-B1- 6 215 519

## Description

### FIELD OF THE INVENTION

This invention relates to the field of face image recordal and identity confirmation using face images and in particular to the means by which faces can be recorded and identity can be confirmed using face images that are automatically obtained (i.e. without human intervention) in security areas where the movement of people cannot be constrained within defined boundaries.

### BACKGROUND OF THE INVENTION

In a world where the prospect of terrorism is an ever increasing threat, there is a need to rapidly screen and record or identify individuals gaining access to certain restricted areas such as airports, sports stadiums, political conventions, legislative assemblies, corporate meetings, etc. There is also a need to screen and record or identify individuals gaining access to a country through its various ports of entry. One of the ways to identify such individuals is through biometric identification using face recognition techniques, which utilize various measurements of a person's unique facial features as a means of identification. Some of the problems associated with using face recognition as a means of rapidly screening and identifying individuals attempting to gain access to a security area are the slow speed of image acquisition, the poor quality of the images acquired, and the need for human operation of such systems.

Attempts to solve these problems in the past have employed a single high-resolution video camera which is used to monitor a security area leading to an entrance. Typically, a fixed focal length lens is employed on the camera. Software is used to analyse the video image to detect and track face images of targets entering the security area. These images are captured, recorded and sent to face recognition and comparison software in an attempt to identify the individuals and verify their right to access the area. One of the main problems with such systems is that the video data is of low resolution and too "noisy" to provide consistently good results. Such systems work reasonably well only when the security area is small and the distances between targets entering the security area and the monitoring camera are relatively constant. Widening the security area and/or trying to accommodate targets at varying distances to the camera, results in some targets having too little resolution in the video image to be properly analysed for accurate face recognition. The main drawback of such systems, therefore, is that they operate successfully only over a very narrow angular and depth range. Captured image quality and therefore the success of face recognition on those images is inconsistent.

Other existing systems use two cameras, one stationary wide field of view camera to monitor the security area and detect faces, and a second, narrow field of view, steerable camera to be pointed, by means of pan, tilt and zoom functions, at the faces identified by the first camera for the purposes of capturing a face image and sending it off for face recognition and comparison to a database. In this method, the second camera is able to obtain high-resolution images necessary for accurate face recognition. The main drawback of these systems is that, as the distance from the first camera increases, it becomes difficult to recognize that a target within the field of view contains a face. Second, the motorized pan, tilt and zoom functions of the second camera are relatively slow. As a result, the system is only capable of tracking one person at a time.

Document US 6122445 discloses a camera associated with a separate range detecting device.

Another solution is to use motorized pan, tilt and zoom cameras, remotely controlled by a human operator to monitor a security area. Such systems are routinely employed to monitor large areas or buildings. A multitude of cameras can be used and normally each operates in a wide-angle mode. When the operator notices something of interest he/she can zoom in using the motorized controls and obtain an image of a person's face for purposes of face recognition. The drawback of such systems is that they require the presence of an operator to detect and decide when to obtain the face images. Such a system is typically so slow that not more than one person can be tracked at a time.

Yet another solution is to require persons seeking entry to a secure area to pass single file, at a restricted pace, through a monitoring area, much the same as passing through a metal detector at an airport. A single, fixed focus camera is set up at a set distance to capture an image of the person's face for face comparison and face recognition. Such a system would severely restrict the flow of persons into the secure area, and in many cases, such as sports stadiums, would be totally unworkable. Moreover, the system would still require an operator to ensure that the camera is pointed directly at the person's face, and do not include any means for ensuring that a proper pose is obtained.

From the above, it is clear that there is a need for an automated face imaging system that overcomes the disadvantages of the prior art by providing the ability to rapidly capture and record high quality face images of persons entering a security area and optionally to make those images available for face comparison and identification. It would be advantageous if such a system included an automated, highly accurate, rapid face detection and face tracking system to facilitate face image capture for the purposes of recordal and/or face comparison and face recognition.

### BRIEF SUMMARY OF THE INVENTION

An object of one aspect of the present invention is to overcome the above shortcomings by providing a face imaging system to rapidly detect face images of target persons within a security area and capture high quality images of those faces for recordal and/or for use in face recognition systems for purposes of face verification, face recognition, and face comparison.

An object of another aspect of the invention is to provide a camera system for a face imaging system that is capable of tracking multiple target faces within a security area and providing high quality images of those faces for recordal and/or for use in face recognition systems for purposes of face verification, face recognition, and face comparison.

An object of a further aspect of the invention is to provide a face imaging system that can provide face images of sufficient size and resolution, in accordance with the requirements of known face recognition and face comparison systems, to enable those systems to function at peak efficiency and to provide consistently good results for face identification and comparison.

An object of still another aspect of the invention is to provide a face imaging system that utilizes range data from a ranging unit or other device and video image data to assist in face image detection and face image tracking.

An object of yet another aspect of the invention is to provide a face imaging system that utilizes a historical record of range data from a ranging unit or other device to assist in face image detection and face image tracking.

According to one aspect of the present invention then, there is provided a face imaging system for recordal and/or automated identity confirmation of a target within a security area, the face imaging system comprising: a camera unit, comprising: a camera unit controller, a ranging unit for detecting the presence of the target within the security area and for providing range data relating to the target to the camera unit controller, and a video camera for capturing images of the target and sending the target images to the camera unit controller, the camera unit controller comprising: a face detection system configured to detect a face image within the target images; a face capture system configured to capture the face image when the face image is determined to be of a sufficient quality for face recognition.

The video camera may itself, either wholly or partially, be actuated to effect tracking of a target, for example, by pan, tilt and focus. Or the video camera may view the scene through an actuated reflector means, for example, a mirror, that can rapidly shift the field of view. The pointing of the camera may also be assisted, at least initially, by range data provided by a presence sensor.

The rate of capture of images is based upon the time spent in each of the specific steps of image detection, image tracking and, finally, image capture. The decision to effect image capture is based upon the presence of an image that meets a predetermined quality threshold. Once image capture has occurred, the system is released to repeat the cycle. One object of the invention is to minimize the cycle time.

In preferred embodiments, the face imaging system described herein uses a high resolution, steerable video camera and a high-resolution laser-based rangefinder. The rangefinder scans the monitored security area, typically with a field of view of 45 degrees approximately every 100 milliseconds and notes the angular locations, distances and widths of any potential targets located therein. The depth of the monitored security area is typically 15 metres but can be modified to suit the particular installation. The angular locations, distances and widths of targets within the monitored security area are presented to a camera unit controller computer that processes the data and sends commands to point the video camera at targets of interest. The commands are for the pan, tilt and zoom functions of the video camera. Based on the distance to the target, the zoom function of the video camera is activated to the degree required to obtain a video image of an average human face filling at least 20% of the image area. Face detection software, assisted by range data specifying the distance, angular location and width of a potential target, is used to analyse the image and determine if it contains a human face. If a face is detected, coordinates of the major face features are calculated and used by the video camera to further zoom in on the face so that it fills almost the entire field of view of the video camera. These coordinates, with reference to the range data and the video image, are constantly updated and can also be used to facilitate the tracking of the target face as it moves about. Once the image quality of the face is determined to be sufficient, according to predetermined criteria based on the face recognition systems being used, face images are captured and recorded and/or made available to face recognition software for biometric verification and identification and comparison to external databases.

The video camera used in the present invention is of a unique design that permits a high speed, accurate pointing operation. The ability of the present invention to rapidly point the video camera enables the tracking of many persons within the security area at the same time in a true multiplexed mode. The video camera is able to point quickly from one person to another and then back again. Unlike other motorized pan, tilt and zoom video cameras, the video camera of the present invention is not moved on a platform to perform the panning operation. Instead, a lightweight mirror is mounted directly on a linear, moving coil, motor and is used to direct an image of a segment of the security area to the video camera. By moving the mirror, the field of view of the video camera can be panned rapidly across the security area in a very brief time, on the order of tens of milliseconds, enabling the system to operate in a true multiplexed mode. Tilting is still performed by moving the video camera itself, but at normal operating distances, the angles over which the video camera must be tilted to acquire a face image are small and can be easily accommodated by existing tilt mechanisms. Zooming is also accomplished in the standard manner by moving the video camera lens elements.

The system of the invention may incorporate image analysis logic that is either "on board" at the location of the camera unit or is situated at a remote location. Thus the camera system can be programed to obtain additional images of special individuals. Face tracking data from the video image may be used to enhance the performance of the face recognition logic operations. Image data can be combined with data from a presence sensor to ensure good lighting and pose control. This can enhance identity confirmation and/or allow the system to maintain a preset standard of consistency.

The benefits of the approach described herein are many. Damage to the video camera is eliminated as it no longer has to be moved quickly back and forth to pan across the security area. Associated cabling problems are also eliminated. No powerful panning motor or associated gears are required to effect the rapid movement of the video camera, and gearing-backlash problems are eliminated. The use of target range data along with target video data allows the system to more accurately detect and track faces in the security area and allows the tracking of multiple target faces. Video and range data is used in a complementary fashion to remove ambiguity inherent in face detection and tracking when only a single source of data is available. Current face recognition software algorithms suffer when the input images are poorly posed, poorly lit, or poorly cropped. The use of target range data in conjunction with target video data allows a more accurate selection of correctly centred images, with good lighting and correct timing of image capture to ensure correct pose. The improved image quality significantly improves face recognition performance.

Further objects and advantages of the present invention will be apparent from the following description and the appended drawings, wherein preferred embodiments of the invention are clearly described and shown.

### BRIEF DESCRIPTION OF THE DRAWINGS

The present invention will be further understood from the following description with reference to the drawings in which:
Figure 1 is a top-down plan view of the present invention installed within a security area.
Figure 2 is a side elevation view showing one possible installation position of the video camera, rotatable mirror system, and the ranging unit of the present invention within a security area.
Figure 3 is a block diagram of the camera unit of the present invention shown in Figure 1.
Figure 4 is a block diagram showing the network architecture of the present invention including multiple camera units and an external controller.

### DETAILED DESCRIPTION OF THE PREFERRED EMBODIMENTS OF THE INVENTION

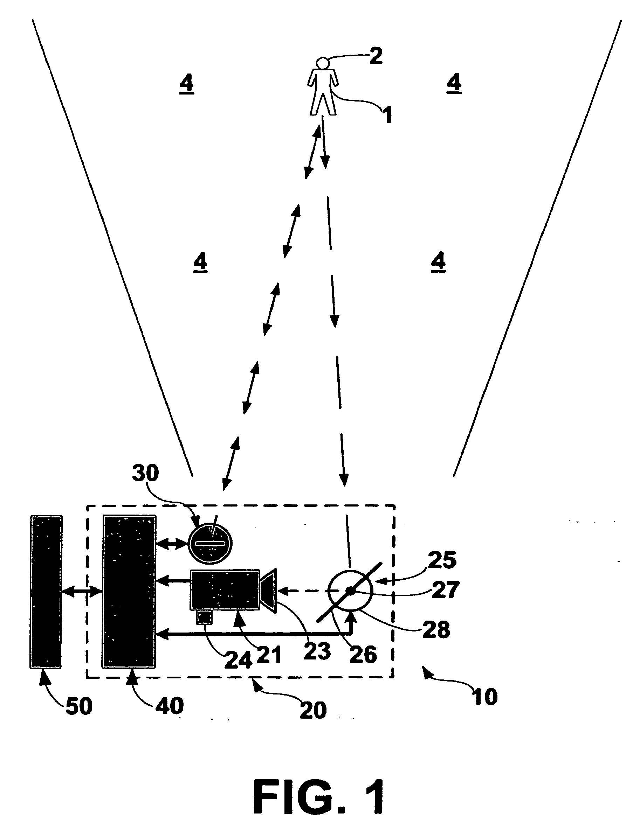

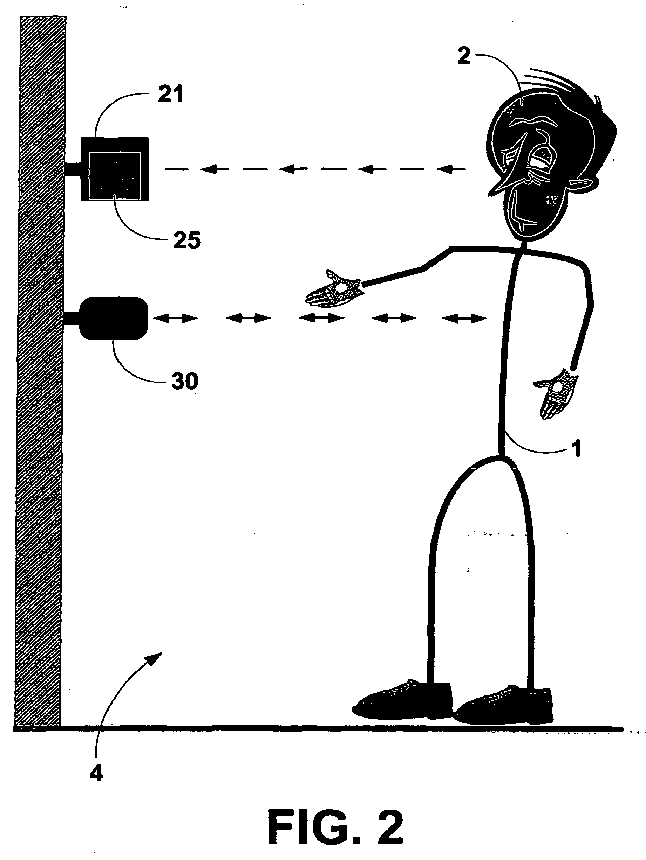

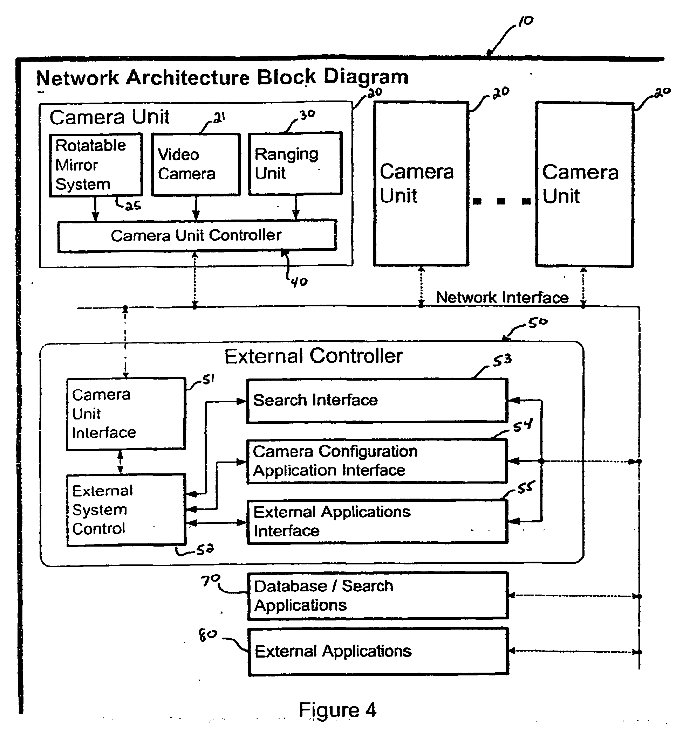

Referring to Figures **1** and **2,** an automated identity confirmation system **10** is shown for monitoring targets **1** and obtaining images of target faces **2** entering a security area **4.** As also shown in the architecture block diagram in Figure **4**, the automated identity confirmation system **10** comprises one or more camera units **20** and an external controller **50**. The camera units **20** include a video camera **21**, a rotatable mirror system **25**, a ranging unit **30**, and a camera unit controller **40**.

It will be understood throughout this discussion that security area **4** is a three-dimensional space. The vertical direction is measured from the bottom to the top of the security area in the normal manner, while from the view point of camera unit **20**, the horizontal direction is measured from side to side, and the depth is measured from camera unit **20** outward, also in the normal manner. Thus, for a person standing within security area **4** and facing camera unit **20**, the vertical direction is from the person's feet to the person's head, the horizontal direction is from the left side of the person to the right, and the depth is from the person's front to back.

### Camera Unit - Video Camera

The camera unit **20** includes a standard video camera **21** of the type frequently used in machine vision systems. Although there are a number of camera models, manufactured by different companies, that would be suitable, in the particular instance described herein, the applicant has used a colour video camera manufactured by Sony™, model number EVI-400. This camera features zoom capability, automatic exposure control and automatic focusing. Video camera **21** includes a video output for sending video signals to camera unit controller **40** and a serial input/output (I/O) interface for connecting to camera unit controller **40** to control and monitor the various camera functions such as zoom, focus and exposure. To extend the range over which video camera **21** operates, a teleconverter lens **23** has been added to enable the capture of an image of a human face **2** at a maximum range in such a manner that the face fills the entire video image. In the present instance, the maximum range has been arbitrarily set at **15** meters, however, by increasing the sensitivity of ranging unit **30** and extending the focal length of lens **23**, the maximum range can be extended. Camera unit **20** includes a tilt motor **24**, and tilt motor driving electronics, for tilting video camera **21** up and down to sweep in the vertical direction. The degree to which video camera **21** needs to be tihed in the vertical direction is small, as it is only necessary to compensate for differences in the vertical height of a person's face from a common reference point, which normally is the average human eye level.

As noted above, camera unit **20** includes focus, tilt and zoom capabilities that permit rapid movement of video camera **21** to acquire high quality face images from target **1.** These features are controlled by camera control signals from camera unit controller **40** through the serial interface. Focus on a particular target selected by ranging unit **30** is automatic and merely requires that video camera **21** point to a target. Zoom is controlled to a setting that will initially permit the field of view of video camera **21** to be substantially larger than what an average human face would represent at the target distance. Typically, the zoom is set so that the average human face would fill 20% of the field of view. Zoom is refined by further signals from camera unit controller **40** based on data from ranging unit **30** and video camera **21.** In the present setup, the tilt function is provided by external tilt motor **24** mounted to video camera **21,** but may in other configurations be incorporated as part of video camera **21.** The amount of tilt required to obtain a high quality face image of target **1** is based on data from ranging unit **30** and video camera **21** and is controlled by signals from camera unit **controller 40.** Range data is important, since target distance is helpful in determining the amount of tilt required.

Where the field of view of video camera **21** is rectangular, having one dimension longer than the other, the applicant has found it advantageous to orient the video camera so that the longer dimension of the field of view is parallel to the vertical direction of security area **4,** thus increasing the capture area for vertical targets, such as persons, within security area **4.** By increasing the capture area for vertical targets, the applicant reduces the amount of video camera tilt required to obtain a high quality face image of the target.

### Camera Unit - Rotatable Mirror System

Camera unit **20** includes a rotatable mirror system **25** located directly in front of video camera **21** as shown in Figure **1.** Rotatable mirror system **25** includes a lightweight mirror **26** mounted directly on a vertical motor shaft **27** of a linear motor **28.** Linear motor **28** is of the type used in computer hard drives, and includes servo electronic drivers sufficient to rotate mirror **26** rapidly and accurately to the intended position. Also included, is a standard positional feedback system, mounted directly on shaft **27,** comprising circuitry which reads the exact position of mirror 26 and outputs a position feedback signal to the servo drivers. By matching the position feedback signal to a command signal received from camera unit controller **40,** representing the intended position of mirror **26,** motor **28** can drive mirror **26** to point directly at the intended location.

In the setup shown in Figures **1** and **2,** ranging unit **30** determines the distance (depth), angular position and width of target **1** within security area **4** and provides those coordinates to camera unit controller **40.** Camera unit controller **40** sends a mirror command signal to mirror system **25,** to cause linear motor **28** to rotate mirror **26** to the proper location, thus providing a horizontal panning feature for camera unit **20.** The image of target **1** incident on mirror **26** is directed to video camera **21** for image capture. By rapidly rotating mirror **26,** video camera **21** can be effectively panned across the entire horizontal extent of security area **4** in a fraction of the time it would take a standard video camera, with a motor driven horizontal pan feature to accomplish the same task. The response time is such that panning from any target within security area **4** to any other target within security area **4** can be accomplished in less than **100** milliseconds. Panning accuracy can be attained to within one-tenth of a degree.

Mirror system **25** may include a mirror brake (not shown), which holds and locks mirror **26** in place once the desired target **1** has been acquired. The mirror brake prevents vibrations in mirror **26,** thereby improving image stability, and thus enhancing image quality. In a preferred embodiment, the mirror brake is an electromagnet located on shaft **27.**

Mirror system **25** could be adapted to include a second degree of rotatable freedom to also provide video camera **21** with a vertical tilt feature, replacing the tilt feature provided by external tilt motor **24.** In the alternative, a second rotatable mirror system could be provided that would include a second mirror, rotatable on an axis positioned at 90 degrees to the axis of rotation of mirror **26.** In combination, the two rotatable mirror systems would provide video camera **26** with both vertical tilting and horizontal panning features

### Camera Unit - Camera/Mirror System Control

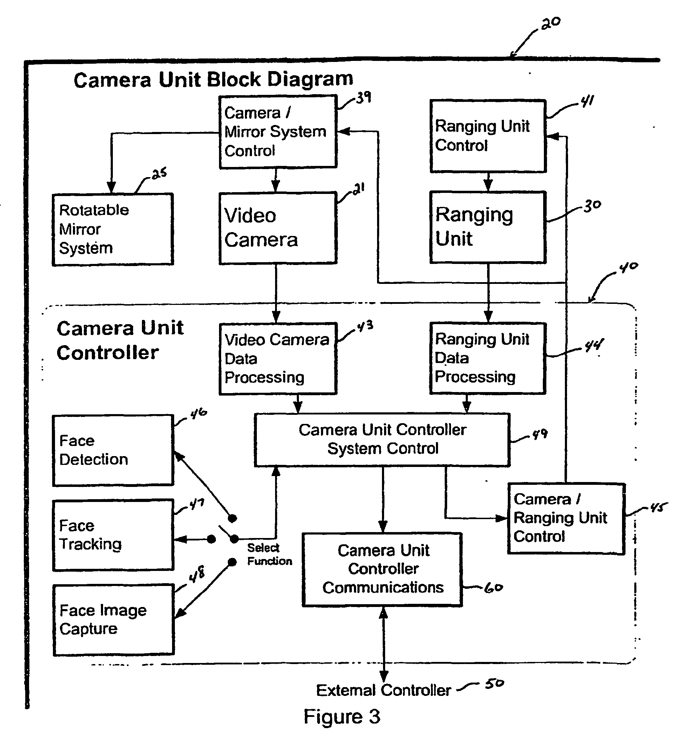

Referring to Figure **3,** a camera/mirror system control **39** is connected to video camera **21** and rotatable mirror system **25** and comprises hardware and software components for receiving camera and mirror system control commands from camera unit controller **40** and for controlling the various functions of video camera **21** and mirror system **25.** These functions include exposure, zoom, focus, tilt, pan (mirror rotation), on/off, video camera frame rate, brightness and contrast. Camera/mirror system control **39** is also responsible for reading back the status of various video camera **21** and mirror system **25** functions and reporting same to camera unit controller **40.**

### Camera Unit - Ranging Unit

Referring to Figures **1 - 4,** camera unit **20** includes a ranging unit **30** to locate targets **1** within security area **4.** In one aspect of the invention, ranging unit **30** is of a common well known design, using a laser diode based distance measuring device operating in conjunction with a rotating range mirror and a range lens receiving system to scan security area **4.** A time-of-flight principle is used to calculate the distance to target **1.** In the present configuration, the laser diode is pulsed, for a period on the order of **10** nanoseconds, once during every 1/4 degree of rotation of the range mirror. The laser beam is reflected by the rotating range mirror into security area **4** and any return pulse reflected by target **1** is measured by the range lens receiving system. Knowing the constant value for the speed of light and the time interval between the emission of the laser pulse and the return reflection, the distance to target **1** can be calculated. Ranging unit **30** records the distance (depth), angular position and width of the detected target within area **4** and sends this information to camera unit controller **40**. Because range unit **30** is capable of recording range data for every 1/4 degree, range data can provide an target profile that can be analyzed to determine whether it matches the profile of a person (relatively smooth). A complete scan of security area **4** can be accomplished during each rotation of the range mirror which occurs every 100 milliseconds, thus permitting extremely rapid detection and location of targets therein. The scanning rate of area **4** is referred to as the ranging unit frame rate and can be varied according to requirements of the installation or mode of operation.

Ranging unit **30** is generally located below video camera **21** at a level equal to the average person's chest height. Video camera **21** is generally located at the average person's eye level. However, other arrangements for ranging unit **30** and video camera **21** are possible depending on the particular installation.

It will be understood by the reader, that other configurations for ranging unit **30** could be used in the present invention. For example, a sonar-based ranging system could be employed, or one based on high frequency radar or binocular/differential parallax.

### Camera Unit - Ranging Unit Control

Ranging unit **30** includes a ranging unit control **41** comprising hardware and software components to manage the various functions of ranging unit **30,** including maintaining range mirror rotation speed within specified parameters, regulating laser diode power saving modes, including a "sleep" mode where the laser pulse rate is reduced during "dead" times when there is no activity in the security area, accepting control functions from camera unit controller **40,** and sending status information regarding ranging unit **30** to camera unit controller **40** on request. Ranging unit control **41** pre-processes range data by performing various functions including, noise filtering functions to eliminate scattered data comprising single unrelated scan points, moving averaging and scan averaging over multiple scan lines to smooth the range data, sample cluster calculations to determine if detected objects represent targets of interest having the necessary width at a given distance, extracting coordinate information from targets of interest in the form of angle, radius (distance) and width, and building a vectorized profile from the range data of each target. Ranging unit control hardware **41** sends either the raw range data, or the preprocessed, vectorized range data profile to camera unit controller 40 for further processing. The vectorized range data is sent in the form n(a1, r1, w1)(a2, r2, w2)..., where n represents the number of targets in the security area scanned, ax represents the angular location of target number x within the security area, rx represents the radius (distance) to target x, and wx represents the width of target x. Range data is sent to camera unit controller **40** on request from range unit control **41** or in a continuous mode at a selectable (programmable) refresh rate.

### Camera Unit - Camera Unit Controller

Camera unit **20** also includes a camera unit controller **40** as shown in greater detail in the block diagram of Figure **3.** Camera unit controller **40** includes all of the hardware and software to provide an interface between video camera **21,** ranging unit **30,** rotatable mirror system **25,** and external controller **50.** The purpose of camera unit controller **40** is to control the detection, tracking and capture of high quality video images of faces **2** of targets **1** of interest within security area **4.** This is accomplished by processing input data received from ranging unit **30** and video camera **21** and using this data to calculate the appropriate pointing command signals to send back to video camera **21** and rotatable mirror system **25.** This is described in greater detail below when discussing the various components of camera unit controller **40**. Camera unit controller **40** also interfaces with external controller **50** to receive external control commands and send captured video images. External control commands are used both to configure the components of camera units **20** and to modify their behavior, for example, to lock onto and track a particular target within security area **4.**

### Camera Unit - Camera Unit Controller Hardware

Camera unit controller **40** includes hardware comprising a computer with CPU, RAM, and storage, with interface connections for video input, serial interfaces and high speed I/O, and Ehternet interface. The output from video camera **21** is received on the video input. The output from and control signals to ranging unit **30** are received on one of the serial ports. Control signals for video camera **21** and rotatable mirror system **25** are sent on one of the other of the serial ports. The network interface is used to connect with external controller **50.** Other hardware configurations are possible for camera unit controller **40,** for example, multiple, low-power CPUs could be used rather than a single high power CPU, the video input from video camera **21** could be a direct digital, or the interface to external controller **50** could be high-speed serial or wireless network, rather than Ehternet.

### Camera Unit - Camera Unit Controller Software

Camera unit controller **40** includes camera unit controller software including a modern network capable multi-tasking operating system to control the operation and scheduling of multiple independent intercommunicating software components. The camera unit controller software components include: video camera data processing **43;** ranging unit data processing **44;** camera/ranging unit control **45;** face detection **46;** face tracking **47;** face image capture **48;** camera unit controller system control **49** and camera unit controller communications **60.**

Video frames arriving from video camera **21** are asynchronously digitized in a hardware video capture board. This data is presented to video camera data processing **43** which comprises software to perform basic image processing operations to normalize, scale and correct the input data. Corrections are made for image colour and geometry based on standard calibration data. Image enhancement and noise filtering is performed, and the processed video image data is made available to the camera unit controller system control **49** where it is used in performing a number of functions including face detection, face tracking, or face image capture (see below).

Range data arrives at camera unit controller **40** from ranging unit **30** either continuously or in response to a request from camera unit controller **40.** The range data takes the form of a table of values of distance (depth or radius), angle and width. The range data is processed by ranging unit data processing **44** which comprises software to determine the position and location of targets **1** within security area **4.** Heuristic methods are used to subtract background and remove small diameter "noise", leaving only larger objects of a size similar to the intended targets, which are persons. These heuristics are intelligent software modules that use historical, probability and statistical analysis of the data to determine the characteristics of objects within the security area. For example, if an object was detected in only one scan of ranging unit **30** and not in the previous or subsequent scans, it can safely be assumed that a spurious event occurred which can be ignored. Similarly, limits can be set on the speed of objects moving in the security area. If an object moved five meters between scans it can safely be assumed that the object is not a person. In addition, calibration data, taken on installation, when security area **4** is totally empty, is used to separate potential targets from fixed objects in the security area, such as support poles and the like (background removal).

The processed range data is made available to camera unit controller system control **49** where it is used to assist in face detection and face tracking. Ranging unit data processing **44** maintains a history buffer of previous range data for each target **1** within security area **4** for a predetermined time interval. The history buffer is used by face detection **46** and face tracking **47** to assist in face detection and face tracking. For example, a single large object may be one large person, or it may be two persons standing close together. If the faces of the two persons are close together it may be difficult to distinguish between the two situations. However, using the history buffer data, it is possible to determine that two single smaller persons were previously separate targets and had moved together. Thus, ambiguous data received from ranging unit 30 and video camera **26** can be clarified.

Camera/ranging unit control **45** comprises software to manage all signals sent via the camera unit controller serial I/O ports to video camera **21,** ranging unit **30** and rotatable mirror system **25.** These control commands go to ranging unit control **41** and camera/mirror system control **39,** and are based on input received from camera unit controller system control **49.** Positional changes of the target, based on changes in range data from ranging unit **30** and on changes in the geometric shape of the target video image from video camera **21**, are determined by camera unit controller system control **49.** Control commands to control video camera on/off; video camera focus; video camera tilt; mirror rotation (panning); video camera zoom; video camera frame rate; video camera brightness and contrast; ranging unit on/off; and ranging unit frame rate, are sent via camera/ranging unit control **45** to facilitate both face detection and face tracking. The purpose of the command signals is to ensure that the target is properly tracked and that a high quality video image of the target's face is obtained for the purpose of face recognition. In addition, camera/ranging unit control **45** manages the appropriate timing of commands sent out, ensures reliable delivery and execution of those commands, alerts camera unit controller system control **49** of any problems with those commands or other problem situations that might occur within video camera **21,** ranging unit **30** or rotatable mirror system **25.** For example, if rotatable mirror system **25** is not responding to control commands it will be assumed that motor **28** is broken or mirror **26** is stuck and an alarm will be sent out to signal that maintenance is needed.

Face detection **46** comprises software to detect face images within the video image arriving from video camera **21.** Initially, face detection **46** uses the entire input video image for the purpose of face detection. A number of different, known software algorithmic strategies are used to process the input data and heuristic methods are employed to combine these data in a way that minimizes the ambiguity inherent in the face detection process. Ambiguity can result from factors such as: variations of the image due to variations in face expression (non-rigidity) and textural differences between images of the same persons face; cosmetic features such as glasses or a moustache; and unpredictable imaging conditions in an unconstrained environment, such as lighting. Because faces are three-dimensional, any change in the light distribution can result in significant shadow changes, which translate to increased variability of the two-dimensional face image. The heuristics employed by face detection **46** comprise a set of rules structured to determine which software algorithms are most reliable in certain situations. For example, in ideal lighting conditions, bulk face colour and shape algorithms will provide the desired accuracy at high speed. Range data from ranging unit **30** is added to narrow the search and assist in determining the specific areas of the video image most likely to contain a human face based on target width and historical movement characteristics of targets within security area **4.**

The following are some of the software algorithms, known in the field, that are used by the applicant in face detection:
Bulk face colour and shape estimation;
Individual face feature detection (for eyes, nose, mouth, etc.) using geometrical constraints to eliminate improbable features;
Artificial neural network analysis based on training the algorithm on a large set of face and non-face data; and
Bayesian analysis using Principle Component Analysis (PCA), or Eigenface decomposition of the face image.

The following additional steps are performed by face detection **46** of the present invention, which utilize range data from ranging unit **30** and have been found by the applicant to increase the ability of the present invention to detect a face within the video image:
Analysis of the range data to isolate person-size targets. As discussed above, this includes intelligent software modules using historical, probability and statistical analysis of the range data to determine the characteristics of objects within the security area and to eliminate noise resulting from small or fast moving objects that are not likely persons. Range data from ranging unit **30** can be used to determine targets of an appropriate width (30 cm to 100 cm) and shape (smooth front surface). Knowing exactly where the person-size target is located within the video image provides a starting point for commencing face detection.
Analysis of the range data history to determine the presence of groups of people. This is done by isolating person-sized targets in each video frame using the above-described technique based on an analysis of the range data. Motion estimation software, such as Kalman filtering, is used to estimate the trajectory of such targets and identify ambiguous targets as those fitting poorly to the Kalman trajectory estimation. Finally, ambiguous targets are classified and the classification is used to assist in face detection. For example, it will be possible to determine whether a particular ambiguity is the result of two or more persons standing close together.

In a preferred embodiment of the invention, face detection 46 identifies an image as corresponding to a face based on colour, shape and structure. Elliptical regions are located based on region growing algorithms applied at a coarse resolution of the segmented image. A colour algorithm is reinforced by a face shape evaluation technique. The image region is labelled "face" or "not face" after matching the region boundaries with an elliptical shape (mimicking the head shape), with a fixed height to width aspect ratio (usually 1.2).

In a further preferred embodiment of the invention, a method of eye detection using infrared (IR) illumination can be used to locate the eyes on a normal human face and thus assist in face detection **46.** In this method, the target is illuminated with bursts of infrared light from an IR strobe, preferably originating co-axially or near co-axially with the optical axis of video camera **21.** The IR increases the brightness of the pupil of the human eye on the video image. By locating these areas of increased brightness, face detection **46** is able to quickly identify and locate a potential face within the video image. If the IR strobe is flashed only during specific identified video frames, a frame subtraction technique can be used to more readily identify areas of increased brightness, possibly corresponding to the location of human eyes. Accurately identifying the location of the eyes has a further advantage, in that such information can greatly improve the accuracy of facial recognition software.

Face detection is intrinsically a computationally intensive task. With current processor speeds, it is impossible to perform full-face detection on each arriving video image frame from video camera **21.** Therefore, the face detection process is only activated by camera unit controller system control **49** when required, that is when no face has been detected within the arriving image. Once a face is detected, face detection is turned off and face tracking **47** takes over. The quality of face tracking **47** is characterized by a tracking confidence parameter. When the tracking confidence parameter drops below a set threshold, the target face is considered lost and face detection resumes. When the tracking confidence parameter reaches a predetermined image capture threshold face images are acquired by face image capture module **48.** Once a sufficient number of high quality face images are acquired, the target is dropped and face detection resumes on other targets.

Once a face is detected within the video image, face tracking **47,** comprising face tracking software, is activated and processes data input from video camera data processing **43** and ranging unit data processing **44** for the purpose of determining the rate and direction of movement of the detected face, both in the vertical, horizontal and depth directions. Face tracking **47** is initialized with the detected target face position and scale and uses a region-of-interest (ROI) limited to the surrounding bounding box of the detected target face. Any movement is reported to camera unit controller system control **49** where it is used to direct the panning of rotatable mirror system **25** and the zoom, focus and tilting functions of video camera **21,** so as to track the target face and keep it within the field of view. The target face is tracked until the tracking confidence drops below a set threshold. In this case the target is considered lost, and the system switches back to detection mode. Camera unit controller system control **49** will determine when to activate face image capture **48.**

Face tracking **47** uses a number of known software algorithmic strategies to process the input video and range data and heuristic methods are employed to combine the results. The heuristics employed comprise a set of rules structured to determine which software, algorithms are most reliable in certain situations. The following are some of the software algorithms, known in the field, that are used by the applicant in face tracking:
Frame to Frame differencing to detect movement;
Optical flow techniques on the video stream;
Bulk face colour and shape estimation;
Kalman filter analysis to filter present movement and predict future movement from past movement estimation; and
Artificial neural network analysis based on training the algorithm on a large set of video sequences.

The following additional step is performed by face tracking **47** of the present invention, which utilizes range data from ranging unit 30 and has been found by the applicant to increase the ability of the present invention to track a face:
Analysis of range data and range data history. As detailed above, a history buffer of previous range data for each target can be used to determine whether a single large object is one large person, or two persons standing close together, or possibly not a person at all.

In a preferred embodiment of the invention, an elliptical outline is fitted to the contour of the detected face. Every time a new image becomes available, face tracking **47** fits the ellipse from the previous image in such a way as to best approximate the position of the face in the new image. A confidence value reflecting the model fitting is returned. The face positions are sequentially analyzed using a Kalman filter to determine the motion trajectory of the face within a determined error range. This motion trajectory is

Many of the face tracking algorithms rely in part on colour and colour texture to perform face tracking. Due to changes in both background and foreground lighting, image colour is often unstable leading to tracking errors and "lost targets". To compensate for changes in lighting conditions, a statistical approach is adopted in which colour distributions over the entire face image area are estimated over time. In this way, assuming that lighting conditions change smoothly over time, a colour model can be dynamically adapted to reflect the changing appearance of the target being tracked. As each image arrives from video camera **21,** a new set of pixels is sampled from the face region and used to update the colour model. During successful tracking, the colour model is dynamically adapted only if the tracker confidence is greater than a predetermined tracking threshold. Dynamic adaptation is suspended in case of tracking failure, and restarted when the target is regained.

Face tracking **47** is activated by camera unit controller system control **49** only when face detection **46** has detected a face within the video image, and the system operating parameters call for the face to be tracked. These operating parameters will depend on the individual installation requirements. For example, in some situations, a few good images may be captured from each target entering the security area. In other situations, certain targets may be identified and tracked more carefully to obtain higher quality images for purposes of face recognition or archival storage.

Face image capture **48** comprises image capture software which analyses data received from video camera **21** and ranging unit **30** to determine precisely when to capture a face image so as to obtain high quality, well lit, frontal face images of the target. Face image capture **48** uses heuristic methods to determine the pose of the face and best lighting. The correct pose is determined by identifying key face features such as eyes, nose and mouth and ensure they are in the correct position. Lighting quality is determined by an overall analysis of the colour of the face.

In a preferred embodiment, video camera **21** is provided with a programmable spot metering exposure system that can be adjusted in size and location on the video image. Once a face image is located, the spot metering system is adjusted relative to the size of the face image and is centered on the face image. The result is a captured face image that is correctly exposed and more suitable for image analysis and facial recognition and comparison.

Face image capture **48** is activated by camera unit controller system control **49** when a face has been detected by face detection **46,** and the system operating parameters call for a face image to be captured. Parameters affecting image capture include: the number of images required, the required quality threshold of those images, and the required time spacing between images. Image quality is based on pose and lighting and is compared to a preset threshold. Time spacing refers to the rapidity of image capture. Capturing multiple images over a short period does not provide more information than capturing one image over the same time period. A minimum time spacing is required to ensure enough different images are captured to ensure that a good pose is obtained. Once a high quality face image is obtained, it is sent to external controller **50**.

The characteristics of the final captured image are determined in large part by the particular face recognition software algorithms being used. One of the main advantages of the present invention is the ability to adjust system operating parameters to provide high, consistent quality face images so as to achieve accurate and consistent face recognition. For example, it is known that certain face recognition software requires a frontal pose, a minimum pixel resolution between the eyes, and a particular quality of lighting. The present invention can be programmed to only capture images which meet this criteria, and to track a given face until such images are obtained, thus ensuring consistent high quality performance of the face recognition system.

Camera unit controller **40** includes a camera unit controller communication system **60** that interfaces via a network connection to connect camera unit controller 40 to external controller **50** to receive configuration and operating instructions or to send video images or data as requested by external controller **50.**

The following types of configuration and operating instructions are accepted by camera unit controller communications system **60:**
Configuration of parameters for face detection, face tracking and face image capture, such as, how long to follow each target, the number of images to capture, the quality and resolution of images required, the time spacing of images, and how many targets to follow;
Calibration instructions to determine the necessary image correction for lighting conditions within the security area;
Instructions to capture calibration data for ranging unit **30,**
Configuration instructions giving the spatial positioning of camera **21** and ranging unit **30;**
Operating mode instructions to turn on/off, go into "sleep" mode, or go into various operational tracking modes. "Sleep" modes for various components can be useful to extend component life and save power. For example, ranging unit **30** can be instructed to reduce its laser pulse rate to one area scan per second once activity in the security area ceases for a certain period of time. As soon as a target is detected, ranging unit 30 will "wake up" and commence normal scanning. This can significantly extend the life of the laser diode.

Various configurations of camera unit controller communication system **60** are possible. Camera units **20** could intercommunicate amongst themselves; camera units **20** could accept commands from and send data to computers other than external controller **50.** Additionally, different communications infrastructure could be used, such as point to point networks, high speed serial I/O, token ring networks, or wireless networking, or any other suitable communication system.

Camera unit controller system control **49** comprises software that overseas all functions within camera unit controller **40.** All data acquired by video camera data processing **43,** ranging unit data processing **44** and camera unit controller communications system **60** are made available to the camera unit controller system control **49** which determines which of the face detection **46,** face tracking **47,** or face image capture **48** software modules to activate. These decisions are based on particular system requirements such as for example, the number of images required, image quality threshold and image time spacing. Also taken into consideration is the particular operating mode. For example, in one operating mode, only the closest target is followed. In another operating mode, the closest three targets may be followed for three seconds in turn. Operating modes are completely programmable and depend on the particular application.

Camera unit controller system control **49** also determines what commands to send to video camera **21,** rotatable mirror system **25,** and ranging unit **30** to control their various functions. Additionally, any exceptional modes of operation, such as responding to system errors, are coordinated by camera unit controller system control **49.**

Camera unit controller system control **49** combines information from face detection **46** (that indicates the image area is likely a face), with tracking information from face tracking **47** (that indicates the image area belongs to a target that is moving like a person), and with range data from ranging unit data processing **44** (that indicates the image area is the shape of a single person), to select which pixels in the video image are likely to be occupied by faces. To do this, the range data must be closely registered in time and space with the video data. Face tracking accuracy is increased by using a probabilistic analysis that combines multiple measurements of face detection information, face tracking information and range data over time.

Camera unit controller system control **49** used a combination of range and image data, to build a motion history file, storing the trajectories of individual targets within security area **4.** This permits the tracking of individual face targets and the capture of a pre-determined number of face images per person.

### External Controller

Figure **4,** is a block diagram showing the network architecture of the present invention. Multiple camera units **20** are shown connected to external controller **50.** Also shown are database / search applications **70** and external applications **80** connected via a network interface. Figure **4** shows the communication and data flow between the various components of the invention. It will be appreciated that the invention does not require that there be a single network connection between all components. Indeed, many security applications require the use of separate networks for each application. The use of multiple camera units **20** will allow for cooperation between camera units to accomplish tasks such as following a target from one security area to another, or covering a large security area with many potential targets.

External controller **50** comprises a computer with network connectivity to interface with camera units **20,** database / search applications **70,** and external applications **80,** which can provide searching of stored face images and additional sources of data input to the system. For example, an external passport control application can provide images of the data page photograph to external controller **50,** which can be combined and compared with images captured from camera units **20** to conduct automatic face recognition to verify that the face image on the passport corresponds to the face image of the person presenting the passport.

External controller **50** includes software comprising a modem network capable multi-tasking operating system that is capable of controlling the operation of multiple independent intercommunicating software components, including: camera unit interface **51;** external system control **52;** search interface **53;** camera configuration application interface **54;** and external applications interface **55.** All network communications are secured using advanced modem network encryption and authentication technologies to provide secure and reliable intercommunications between components.

Camera unit interface **51** includes software that controls communications with camera unit controllers **40.** Commands are accepted from external system control **52** and sent to camera units **20.** Camera unit interface **51** ensures reliable delivery and appropriate timing of all such communications. Face images arriving from camera units **20** are stored and sequenced to be further processed by other software modules within external controller **50.**

External system control **52** includes software that oversees all functions of external controller **50.** All data acquired by camera unit interface **51,** search interface **53,** camera configuration application interface **54,** and external applications interface **55,** are made available to external system control **52**. Any activities that require coordination of camera units **20** are controlled by external system control **52.** Additionally, any exceptional modes of operation, such as responding to system errors, are coordinated by external system control **52.**

Search interface **53** includes software that provides an interface between external controller **50** and database / search applications **70,** as will be described below, ensuring reliable delivery and appropriate timing of all communications therebetween.

Camera configuration application interface **54** includes software that accepts data input from a camera configuration application. A camera configuration application may be located on external control ler **50** or on another computer located externally and connected via a network. Camera configuration data is used to send commands to camera units **20** to control various operational and configuration functions, such as exposure, colour mode, video system, etc., to instruct camera units **20** to take calibration data, or shift into operational mode and commence following a specific target.

External applications interface **55** includes software that provides an interface between external controller **50** and external applications **80,** as will be described below, ensuring reliable delivery and appropriate timing of communications therebetween.

### Database / Search Applications

Database / search applications **70** is a general term use to describe all of the various search functions that can inter-operate with the present invention. These applications accept data from external controller **50,** and possibly from other data sources, such as passport control applications, to perform searches, and return a candidate list of possible matches to the input data.

Examples of database / search applications include, but are not limited to:
Face Verification: a captured face image received from camera unit **20** is compared to a face image taken from a presented identification document, such as a passport or other picture identification document. Face recognition and comparison software is engaged to determine whether or not there is a match and the results are returned for a report.
Face Identification: face images received from camera units **20** are compared against an alert or "look out" list, containing undesirables. A candidate list of zero or more possible matches is returned for a report.
Database search: identification data from an identification document such as name, identification number, gender, age, and nationality is compared against an alert list. A candidate list of zero or more possible matches to the alert list is returned for a report.

### External Applications

External applications **80** is a general term used to describe other possible security identification systems that are monitoring the same targets or security area as the present invention described herein. Data from external applications **80** can be input to the present system to enhance functionality. It will be appreciated that the details of the interaction between the present invention and external applications **80** will depend on the specific nature of the external applications.

One example of an external application is a passport control system. Travellers present identification documents containing identification data and face images to passport control officers. Identification data and face images from the identification documents are input through external controller **50** to provide enhanced functionality, especially in database / search applications. For example, an image of the traveller obtained from the identification documents can be compared to images of the traveller captured by camera unit **20** to ensure a match (verification). In another example, identification data from the identification document such as gender, age, and nationality can be used to filter the candidate list of face images returned by a face recognition search of the captured face image from camera unit **20** against an alert database.

Additionally, external controller **50** can send information gathered from camera units **20** to external applications **80** to allow enhanced functionality to be performed within these applications. For example, face images captured from camera units **20** can be sent to a passport control application to provide the passport control officer with a side-by-side comparison with the face image from a traveller's identification document. In another example, face images from camera units **20** can be used to allow database search applications to begin processing prior to presentation of identification documents to a passport control officer.

### Setup and Calibration

Referring to Figure **2,** in a typical installation, ranging unit **30** is setup to scan security area 4 horizontally at approximately chest height for the average person. Video camera **21** and rotatable mirror system **25** are positioned at approximately eye level for the average person so that the field of view of video camera **21** covers security area **4.** The exact positions of ranging unit **30**, video camera **21,** and rotatable mirror system **25** are accurately measured and their positions within security area **4** are input to camera unit controller **40** as calibration data. Optionally, as shown in Figure **4,** many camera units **20** can be used to cover a large security area, or multiple related areas can be monitored. Depending on the nature of the installation and application requirements, adjustments may be required to the mode of operation and the intercommunication protocols between the various system components.

Ranging unit **30** is calibrated by obtaining and storing range data from security area **4** containing no transient targets. Subsequently, range data obtained during operation is compared to the calibration data to differentiate static objects from transient targets of interest. Video camera **21** provides sample images of known targets under existing operating light conditions. These images allow calibration of face detection **46** and face tracking **47.**

### Operation

In operation, ranging unit **30** continuously scans monitored security area **4** to detect the presence of targets. Range data, comprising the angular position, distance and width of any potential targets, is transmitted to camera unit controller **40.** Camera unit controller **40** processes the range data and identifies targets most likely to be persons based on the location of the targets (closest first), size (person size) and movement history. Once a target is identified for closer inspection, commands are sent by camera controller unit **40** to video camera **21** and mirror system **25** causing them to execute pan and zoom functions so as to obtain a more detailed view of the target. These commands cause mirror **26** to rotate so the target is brought into the field of view of video camera **21** and the zoom of video camera **21** is activated in accordance with the measured distance so that the average human face will fill 20% of the field of view. Face detection **46** is engaged and uses data obtained from the video image combined with the range data to execute face detection algorithms to determine if the image from video camera **21.** contains a human face. If a human face is detected, face features are extracted and the spacial coordinates of the centre of the face are calculated. This location information is passed back to camera unit controller system control **49** enabling it to send refined pan (mirror rotation), tilt and zoom commands to video camera **21** and mirror system **25** to cause the detected face to fully fill the video image.

Normally, at this point, camera unit controller **40** will initiate a face tracking mode, to follow the person of interest by using the range and video data to calculate the appropriate pan, zoom, and tilt commands that need to be issued to keep video camera **21** accurately pointed at the target's face and to maintain the desired face image size. While tracking the target, heuristic methods are used to determine appropriate moments to capture high quality, frontal-pose images of the target's face. Also, considered are preset image quality threshold, the number of images required, and the time spacing between images. Once obtained, the images are sent to external controller **50** via a network connection. At this point, camera unit controller **40** will either continue to follow the target, or will shift its attention to tracking another target of interest that may have entered within security area **4,** as determined by the application specific work flow logic.

External controller **50** receives the captured video face images and target movement information from each camera unit **20.** It also receives information from external applications **80** such as passport control software that may be monitoring the same target persons. As noted briefly above, one example of external information is a photo image captured from an identification document presented by a target person. External controller **50** interfaces with face recognition and other database search software to perform verification and identification of target persons.

Additionally, external controller **50** can coordinate operation between multiple cameras units 20 to enable the following functions:
1) Tracking of a single person of interest as they pass from one monitored area to another.
2) Coordination of multiple cameras units **20** monitoring a single room. In this situation, targets of interest are identified and to allow the various camera units 20 for face tracking and face image capture.

### Other Applications

In addition to the above-described applications, other applications of the present invention include, but are not limited to:
1. Capture the face image of a person receiving an identification document such as a passport or visa and storing that image in a database for use at a later time when machine-assisted identity confirmation is required to verify the identity of the person who presents the identity document.
2. Perform a "lookout check" on any person applying for an official identity document by capturing face images of the person and sending those images to a database search application for face recognition, identification and comparison to a database of undesirables.
3. Capture face images of the person picking up an identity document and comparison to the face image of the person on the identity document, to verify that the document is issued to the rightful holder.
4. Capture and store in a database the face image of a person when that person receives approval to travel to or enter a country. Capturing of such face images may or may not be based on a risk profile. The database is then used to compare with face images recorded from detained uncooperative persons, or unauthorized persons entering certain security areas, to determine if the person has been seen before and if so, what identity documents were presented at that time.
5. Capture and store in a database the face images of persons checking in for air travel to create an Advance Passenger Information ("API") database. API records are sent to authorities at the arriving destination where they are used to perform advance lookout checks before the flight arrives to identify any persons who ought to be subject to detailed inspection upon arrival.
6. Use API data gathered in the above example to support automated inspection of passengers at the arrival destination. Face images of arriving passengers are captured and compared to API data to ensure that those persons arriving are the same persons who boarded the plane. This allows a rapid deplaning process where passengers can literally walk-through a security area and those that ought to be subject to detailed inspection can be easily identified and selected.
7. Capture face images of persons boarding any public transportation, such as planes, trains or buses, or when attempting to enter any security area including ports of entry into countries or sports stadiums, and sending those images to a database search application for face recognition, identification and comparison to a database of undesirables, to prevent such undesirables from using the public transportation, or entering the security area.
8. Capture face images of persons checking in for public transportation and comparing those images to a face image contained on an identity document presented during check-in, to verify that the correct person is presenting the identity document.
9. Capturing face images of persons upon approach to a country's port of entry inspection area and sending those images to a database search application for face identification and comparison to a database of undesirables to assist the inspection authority in determining whether the person approaching ought to be allowed entry.
10. Capture face images of persons being processed at self-service inspection machines upon arrival to a country's port of entry and sending those images to a database search application for face identification and comparison to a database of undesirables to prevent entry of such persons into the country.
11. Capture face images of all arriving passengers at all arrival gates and store those images in an arrivals database along with the arriving flight details. Use the arrivals database to compare with face images obtained from persons who appear at inspection counters without proper identification and who refuse to supply flight arrival details. This allows border control authorities to identify the airline and the origin of the person so that the airline can be fined and forced to carry the detained person back to the point of origin.
12. Perform a "lookout check" on any person entering within any security area by capturing face images of the person and sending those images to a database search application for face identification and comparison to a database of undesirables and alerting security.
13. Improve airline check-in procedures by capturing face images of passengers as they approach various security areas and comparing those images to face images of booked passengers. For example, the face image of the traveller can be obtained upon initial booking, or at check-in, and used to verify the identity of the person entering other security areas within the airport and eventually boarding the plane. This can greatly increase the speed of check-in and boarding.
14. Face images of persons boaiding a plane can be compared to face images of persons at check-in to verify that the person who checked in is the same person who boarded the plane and to match that person to luggage loaded on the plane.
15. Face images taken continuously by multiple camera units 20 located in many security areas throughout a given location, such as an airport, can be used to locate any person at any given time. In this way, a passenger who fails to show up for a flight can be located and directed to the appropriate boarding area. Flight delays necessitated to located the wayward passenger can be reduced. Such monitoring systems can also be valuable in a prison environment to locate prisoners.
16. In situations involving financial transactions, such as at automated bank teller machines (ATM), captured face images can be used to compare against data from the ATM card to verify that the correct person is using the card.
17. Capture face images of all persons entering a security area for comparison to a database/search application, to ensure that the person is on a pre-approved list of persons permitted entry.

The above is a detailed description of particular preferred embodiments of the invention. Those with skill in the art should, in light of the present disclosure, appreciate that obvious modifications of the embodiments disclosed herein can be made without departing from the spirit and scope of the invention. All of the embodiments disclosed and claimed herein can be made and executed without undue experimentation in light of the present disclosure. The full scope of the invention is set out in the claims that follow and their equivalents. Accordingly, the claims and specification should not be construed to unduly narrow the full scope of protection to which the present invention is entitled.

## Claims

1. A face imaging system for recordal and/or automated identity confirmation of a target within a security area, said face imaging system comprising:
a camera unit, comprising:
a camera unit controller;
a ranging unit for detecting the presence of the target within the security area and for providing range data relating to the target to said camera unit controller; and
a video camera for capturing images of the target and sending said target images to said camera unit controller,
said camera unit controller comprising:
a face detection system configured to detect a face image within said target images;
a face capture system configured to capture said face image when said face image is determined to be of a sufficient quality fur face recognition.

2. The face imaging system of claim 1, wherein said camera unit controller includes a face tracking system configured to track said face image.

3. The face imaging system of claim 2, wherein said camera unit controller includes video camera data processing software configured to process said target images and to provide video image data and wherein said face detection system and said face tracking system are configured to use said video image data to assist in said detection and said tracking of said face image

4. The face imaging system of any of claims 1 to 3, wherein said camera unit includes a rotatable mirror system for reflecting said target images and said face images to said video camera.

5. The face imaging system of any of claims 1 to 4, wherein said face detection system is configured to use said range data to assist in detecting said face images, said range data comprising a distance and an angular location.

6. The face imaging system of claim 5, wherein said range data includes a width of said target.

7. The face imaging system of claim 2, wherein said face tracking system is configured to use said range data to assist in tracking said faces images, said range data comprising a distance and an angular location.

8. The face imaging system of claim 7, wherein said range data includes a width of said target.

9. The face imaging system of any of claims 1 to 8, including a camera unit communication system for sending said captured face images to an external controller for purposes of face verification and/or face recognition.

10. The face imaging system of any of claims 1 to 9, wherein said face detection system is configured to use colour, shape and the location of structural characteristics in said target images to identify said face image.

11. The face imaging system of any of claims 1 to 10, wherein said face detection system includes individual face feature detection to locate and confirm face features within said target images.

12. The face imaging system of any one of claims 1 to 11, wherein said face detection system includes an eye detection system that uses infrared illumination of said target to locate the position of eyes within said target images.

13. The face imaging system of any of claims 1 to 12, wherein said face detection system is configured to turn off when said face image is detected within said target images.

14. The face imaging system of any of claims 1 to 13, wherein said face capture system includes a pose assessment system configured to assess a pose of said face image, for the purpose of determining if said face image is of said sufficient quality for face recognition.

15. The face imaging system of claim 14, wherein said pose assessment system is capable of determining if said pose of said face image is a frontal pose.

16. The face imaging system of any of claims 1 to 15. wherein said face capture system includes a lighting assessment system configured to assess lighting of said face image, for the purpose of determining if said face image is of said sufficient quality for face recognition.

17. The face imaging system of claim 14, wherein said pose assessment system includes a facial feature identification system and a facial feature locating system.

18. The face imaging system of claim 16, wherein said lighting assessment system includes a colour analysis system.

19. The face imaging system of any of claims 1 to 18, wherein said face capture system includes an exposure system configured to measure an exposure of said face image for controlling said face capture system to determine if said face image is of said sufficient quality for face recognition, said exposure system being based on an area, said area being adjustable in size and location on said face image.

20. The face imaging system of any of claims 1 to 19, wherein said face detection system includes individual face feature detection configured to locate the position of eyes within said target images, and wherein said face capture system includes a pixel resolution assessment system for determining a minimum pixel resolution of said face image between said eyes to determine if said face image is of said sufficient quality for face recognition.

21. The face imaging system of claim 1, wherein said range data is used to assist said face detection system in the detection of said face image.

22. The face imaging system of claim 1, wherein said range data is used to assist said face capture system in the capture of said face image.

23. The face imaging system of claim 2, wherein said range data is used to assist said face tracking system in the tracking of said face image.

24. The face imaging system of claim 1, wherein said camera unit controller includes video camera data processing software configured to proms said target images and to provide processed video image data, and wherein said face detection system is configured to use said processed video image data to assist in said detection of said face image.

25. The face imaging system of claim 1, wherein said camera unit controller includes video camera data processing software configured to process said target images and to provide processed video image data, and wherein said face capture system is configured to use said processed video image data to assist in said capture of said face image.

26. The face imaging system of claim 2, wherein said camera unit controller includes video camera data processing software configured to process said target images and to provide processed video image data, and wherein said face tracking system is configured to use said processed video image data to assist in said tracking of said face image.

27. The face imaging system of any of claims 24, 25 or 26, wherein said processed video image data is organized and stored in a motion history file.

28. The face imaging system of claim 1, wherein said camera unit includes automated horizontal pan, zoom and vertical tilt features.

29. The face imaging system of claim 28, wherein said horizontal pan feature is provided by a first mirror system rotatable in a horizontal direction for reflecting said target images into said video camera.

30. The face imaging system of any of claims 28 or 29, wherein said camera unit controller is configured to use said range data to assist in automatically controlling said horizontal pan, said zoom and said vertical tilt features.

31. The face imaging system of any of claims 28 to 30, wherein said vertical tilt feature is provided by said first mirror system which is also rotatable in a vertical direction.

32. The face imaging system of any of claims 28 to 30. wherein said vertical tilt feature is provided by a second mirror system rotatable in a vertical direction for reflecting said target images into said video camera.

33. The face imaging system of any of claims 28 to 32, wherein said camera unit controller includes video camera data processing software to process said target images and to provide video image data, and wherein said camera unit controller is configured to use said video image data to assist in automatically controlling said horizontal pan, said zoom and said vertical tilt features.

34. The imaging system of any of claims 28 to 33, wherein said face detection system generates face image data and said camera unit controller is configured to use said face image data to assist in automatically controlling said horizontal pan, said zoom and said vertical tilt features.

35. The imaging system of claim 2, wherein said camera unit includes amomated horizontal pan, zoom and vertical tilt features, and wherein said face tracking system generates face tracking data and said camera unit controller is configured to use said face tracking data to assist in automatically controlling said horizontal pan, said zoom and said vertical tilt features.

## Patentansprüche

1. Gesichtsbilderzeugungssystem zur Aufzeichnung und/oder automatisierten Bestätigung der Identität eines Zielobjektes innerhalb eines Sicherheitsbereiches, wobei das Gesichtsbilderzeugungssystem enthält:
eine Kameraeinheit mit:
einer Kameraeinheit-Steuereinrichtung;
eine Bereichsmesseinrichtung zum Feststellen der Anwesenheit des Zielobjektes in dem Sicherheitsbereich und zum Liefern von Entfernungsdaten bezüglich des Zielobjektes an die genannte Kameraeinheit-Steuereinrichtung; und
einer Videokamera zum Einfangen von Bildern des Zielobjektes und Senden der genannten Zielobjektbilder an die genannte Kameraeinheit-Steuereinrichtung;
wobei die Kameraeinheit-Steuereinrichtung aufweist:
ein Gesichtsfeststellungsystem, welches konfiguriert ist, um ein Gesichtsbild innerhalb der genannten Zielobjektbilder festzustellen;
ein Bildeinfangsystem, welches konfiguriert ist, um das genannte Gesichtsbild einzufangen, wenn das genannte Gesichtsbild dahingehend festgestellt wird, dass es eine genügende Qualität für eine Gesichtserkennung hat.

2. Gesichtsbilderzeugungssystem nach Anspruch 1, bei welchem die genannte Kameraeinheit-Steuereinrichtung ein Gesichtsaufspürsystem einschließt, welches konfiguriert ist, um das genannte Gesichtsbild aufzuspüren.

3. Gesichtsbilderzeugungssystem nach Anspruch 2, bei welchem die genannte Kameraeinheit-Steuereinrichtung eine Videaokamera-Datenverarbeitungssoftware einschließt, welche konfiguriert ist, um die genannten Zielobjektbilder zu verarbeiten und Videobilddaten zu liefern, und bei welchem das genannte Gesichtsfeststellungssystem und das genannte Gesichtsaufspürsystem konfiguriert sind, um die genannten Videobilddaten zu verwenden, um bei der genannten Feststellung und dem genannten Aufspüren des genannten Gesichtsbildes zu helfen.

4. Gesichtsbilderzeugungssystem nach einem der Ansprüche 1 bis 3, bei welchem die Kameraeinheit ein rotierbares Spiegelsystem zum Reflektieren der genannten Zielobjektbilder und der genannten Gesichtsbilder zu der genannten Videokamera einschließt.

5. Gesichtsbilderzeugungssystem nach einem der Ansprüche 1 bis 4, bei welchem das genannte Gesichtsfeststellungssystem konfiguriert ist, um die genannten Bereichsmessdaten zu benutzen, um bei dem Feststellen der genannten Gesichtsbilder zu helfen, wobei die Bereichsmessdaten eine Abstandslage und eine Winkellage aufweisen.

6. Gesichtsbilderzeugungssystem nach Anspruch 5, bei welchem die genannten Bereichsmessdaten eine Breite des genannten Zielobjekts einschließen.

7. Gesichtsbilderzeugungssystem nach Anspruch 2, bei welchem das genannte Gesichtsaufspürsystem konfiguriert ist, um die genannten Bereichsmessdaten zu benutzen, um bei dem Aufspüren der genannten Gesichtsbilder zu helfen, wobei die Bereichsmessdaten eine Abstandslage und eine Winkellage aufweisen.

8. Gesichtsbilderzeugungssystem nach Anspruch 7, bei welchem die genannten Bereichsmessdaten eine Breite des genannten Zielobjekts einschließen.

9. Gesichtsbilderzeugungssystem nach einem der Ansprüche 1 bis 8, mit einem Kameraeinheit-Übermittlungssystem zum Senden der genannten eingefangenen Gesichtsbilder an eine externen Steuereinrichtung zu Zwecken einer Gesichtsüberprüfung und/oder Gesichtserkennung.

10. Gesichtsbilderzeugungssystem nach einem der Ansprüche 1 bis 9, bei welchem das genannte Gesichtsfeststellungssystem konfiguriert ist, um Farbe, Gestalt und Lage von strukturellen Charakteristiken in den genannten Zielobjektbildern zu benutzen, um das genannte Gesichtsbild zu identifizieren.

11. Gesichtsbilderzeugungssystem nach einem der Ansprüche 1 bis 10, bei welchem das genannte Gesichtsfeststellungssystem individuelle Gesichtsmerkmalsfeststellung einschließt, um Gesichtsmerkmale innerhalb der genannten Zielobjektbilder zu lokalisieren und zu bestätigen.

12. Gesichtsbilderzeugungssystem nach einem der Ansprüche 1 bis 11, bei welchem das genannte Gesichtsfeststellungssystem ein Augenfeststellungssystem einschließt, welches Infrarotbeleuchtung des genannten Zielobjektes benutzt, um die Position der Augen innerhalb der genannten Zielobjektbilder zu lokalisieren.

13. Gesichtsbilderzeugungssystem nach einem der Ansprüche 1 bis 12, bei welchem das genannten Gesichtfeststellungssystem konfiguriert ist, um abzudrehen, wenn das genannte Gesichtsbild innerhalb der genannten Zielobjektbilder festgestellt ist.

14. Gesichtsbilderzeugungssystem nach einem der Ansprüche 1 bis 13, bei welchem das genannte Gesichtseinfangsystem ein Haltungsbeurteilungssystem einschließt, welches konfiguriert ist, um eine Haltung des genannten Gesichtsbildes zu dem Zweck zu beurteilen, um festzulegen, ob das genannte Gesichtsbild von der genannten hinreichenden Qualität für eine Gesichtserkennung ist.

15. Gesichtsbilderzeugungssystem nach Anspruch 14, bei welchem das genannte Haltungsbeurteilungssystem in der Lage ist, festzulegen, ob die genannte Haltung des genannten Gesichtsbildes eine Frontalhaltung ist.

16. Gesichtsbilderzeugungssystem nach einem der Ansprüche 1 bis 15, bei welchem das genannte Gesichtseinfangsystem ein Beleuchtungsbeurteilungssystem einschließt, welches konfiguriert ist, um ein Beleuchten des genannten Gesichtsbildes zu dem Zweck zu beurteilen, um festzulegen, ob das genannte Gesichtsbild von der genannten hinreichenden Qualität für eine Gesichtserkennung ist.

17. Gesichtsbilderzeugungssystem nach Anspruch 14, bei welchem das genannte Haltungsbeurteilungssystem ein Gesichtsmerkmalidentifikationssystem und ein Gesichtsmerkmallokalisiersystem einschließt.

18. Gesichtsbilderzeugungssystem nach Anspruch 16, bei welchem das genannte Beleuchtungsbeurteilungssystem ein Farbanalysesystem einschließt.

19. Gesichtsbilderzeugungssystem nach einem der Ansprüche 1 bis 18, bei welchem das genannte Gesichtseinfangsystem ein Belichtungssystem aufweist, welches konfiguriert ist, um eine Belichtung des genannten Gesichtsbildes zum Steuern des genannten Gesichtseinfangsystems zu messen, um festzulegen, ob das genannte Gesichtsbild von der genannten hinreichenden Qualität für eine Gesichtserkennung ist, wobei das Belichtungssystem auf einem Bereich basiert, wobei der genannte Bereich in Größe und Lage auf dem genannten Gesichtsbild einstellbar ist.

20. Gesichtsbilderzeugungssystem nach einem der Ansprüche 1 bis 19, bei welchem das genannte Gesichtsfeststellungssystem eine individuelle Gesichtsmerkmalsfeststellung einschließt, welche konfiguriert ist, um die Position der Augen innerhalb der genannten Zielobjektbilder zu lokalisieren, und bei welchem das genannte Gesichtseinfangsystem ein Pixelauflösungsbeurteilungssystem zum Festlegen einer minimalen Pixelauflösung des genannten Gesichtsbildes zwischen den genannten Augen festzulegen, um festzulegen, ob das genannte Gesichtsbild von der genannten ausreichenden Qualität für eine Gesichtserkennung ist.

21. Gesichtsbilderzeugungssystem nach Anspruch 1, bei welchem die genannten Bereichsmessdaten benutzt werden, um dem genannten Gesichtsfeststellungssystem bei der Feststellung des genannten Gesichtsbildes zu helfen.

22. Gesichtsbilderzeugungssystem nach Anspruch 1, bei welchem die genannten Bereichsmessdaten benutzt werden, um dem genannten Gesichtseinfangsystem bei dem Einfangen des genannten Gesichtsbildes zu helfen.

23. Gesichtsbilderzeugungssystem nach Anspruch 2, bei welchem die genannten Bereichsmessdaten benutzt werden, um dem genannten Gesichtsaufspürsystem beim Aufspüren des genannten Gesichtbildes zu helfen.

24. Gesichtsbilderzeugungssystem nach Anspruch 1, bei welchem die genannte Kameraeinheit-Steuereinrichtung Videokamera-Datenverarbeitungssoftware einschließt, welche konfiguriert ist, um die genannten Zielobjektbilder zu verarbeiten und verarbeitete Videobilddaten zu liefern, und bei welchem das genannte Gesichtsfeststellungssystem konfiguriert ist, die genannten verarbeiteten Videobilddaten zu benutzen, um bei der genannten Feststellung des genannten Gesichtsbildes zu helfen.

25. Gesichtsbilderzeugungssystem nach Anspruch 1, bei welchem die genannte Kameraeinheit-Steuereinrichtung Videokamera-Datenverarbeitungssoftware einschließt, welche konfiguriert ist, um die genannten Zielobjektbilder zu verarbeiten und verarbeitete Videobilddaten zu liefern, und bei welchem das genannte Gesichtseinfangsystem konfiguriert ist, um die genannten verarbeiteten Videobilddaten zu benutzen, um bei dem genannten Einfang des genannten Gesichtsbildes zu helfen.

26. Gesichtsbilderzeugungssystem nach Anspruch 2, bei welchem die genannte Kameraeinheit-Steuereinrichtung Videokamera-Datenverarbeitungssoftware einschließt, welche konfiguriert ist, um die genannten Zielobjektbilder zu verarbeiten und verarbeitete Videobilddaten zu liefern, und bei welchem das genannte Gesichtsaufspürsystem konfiguriert ist, um die genannten verarbeiteten Videobilddaten zu benutzen, um bei dem genannten Aufspüren des genannten Gesichtsbildes zu helfen.

27. Gesichtsbilderzeugungssystem nach einem der Ansprüche 24, 25 oder 26, bei welchem die genannten verarbeiteten Videobilddaten in einer Bewegungsablaufdatei organisiert und gespeichert werden.

28. Gesichtsbilderzeugungssystem nach Anspruch 1, bei welchem die Kameraeinheit automatisierte Horizontalschwenk-, Zoom- und Vertikalneigungs-Merkmale einschließt.

29. Gesichtsbilderzeugungssystem nach Anspruch 28, bei welchem das genannte Horizontalschwenkmerkmal von einem ersten Spiegelsystem geliefert wird, welches in einer horizontalen Richtung rotierbar ist, um die genannten Zielobjektbilder in die genannte Videokamera zu reflektieren.

30. Gesichtsbilderzeugungssystem nach einem der Ansprüche 28 oder 29, bei welchem die genannte Kameraeinheit-Steuereinrichtung konfiguriert ist, um die genannten Bereichsmessdaten zu benutzen, um bei dem automatisierten Steuern der genannten Horizontalschwenk-, Zoom- und Vertikalneigungs-Merkmale zu helfen.

31. Gesichtsbilderzeugungssystem nach einem der Ansprüche 28 bis 30, bei welchem das genannte Vertikalneigungs-Merkmal von dem genannten ersten Spiegelsystem geliefert wird, welches auch in einer vertikalen Richtung rotierbar ist.

32. Gesichtsbilderzeugungssystem nach einem der Ansprüche 28 bis 30, bei welchem das genannte Vertikalneigungs-Merkmal von einem zweiten Spiegelsystem geliefert wird, welches in einer vertikalen Richtung rotierbar ist, um die genannten Zielobjektbilder in die genannte Videokamera zu reflektieren.

33. Gesichtsbilderzeugungssystem nach einem der Ansprüche 28 bis 32, bei welchem die genannte Kameraeinheit-Steuereinrichtung Videokamera-Datenverarbeitungssoftware einschließt, um die genannten Zielobjektbilder zu verarbeiten und Videobilddaten zu liefern, und bei welchem die genannte Kameraeinheit-Steuereinrichtung konfiguriert ist, um die genannten Videobilddaten zu benutzen, um bei dem automatisierten Steuern der genannten Horizontalschwenk-, Zoom- und Vertikalneigungs-Merkmale zu helfen.

34. Bilderzeugungssystem nach einem der Ansprüche 28 bis 33, bei welchem das genannte Bildfeststellungssystem Gesichtsbilddaten erzeugt und die genannte Kameraeinheit-Steuereinrichtung konfiguriert ist, um die genannten Gesichtsbilddaten zu benutzen, um bei dem automatisierten Steuern der genannten Horizontalschwenk-, Zoom- und Vertikalneigungs-Merkmale zu helfen.

35. Bilderzeugungssystem nach Anspruch 2, bei welchem die genannte Kameraeinheit automatisierte Horizontalschwenk-, Zoom- und Vertikalneigungs-Merkmale einschließt, und bei welchem das genannte Bildaufspürsystem Gesichtsaufspürdaten erzeugt und die genannte Kameraeinheit-Steuereinrichtung konfiguriert ist, um die genannten Bildaufspürdaten zu benutzen, um bei einem automatisierten Steuern der genannten Horizontalschwenk-, Zoom- und Vertikalneigungs-Merkmale zu helfen.

## Revendications

1. Système d'imagerie faciale destiné à l'enregistrement et/ou la confirmation automatisée d'identité d'une cible à l'intérieur d'une zone de sécurité, ledit système d'imagerie faciale comprenant :
une unité de caméra, comprenant :
un dispositif de commande d'unité de caméra ;
une unité télémétrique destinée à détecter la présence de la cible à l'intérieur de la zone de sécurité et destinée à fournir des données de télémétrie relatives à la cible audit dispositif de commande d'unité de caméra ; et
une caméra vidéo destinée à capturer des images de la cible et à envoyer lesdites images de cible audit dispositif de commande d'unité de caméra ;
ledit dispositif d'unité de caméra comprenant :
un système de détection faciale configuré pour détecter une image de face parmi lesdites images cibles ;
un système de capture faciale configuré pour capturer ladite image de face lorsque ladite image de face est déterminée comme étant d'une qualité suffisante pour une reconnaissance faciale.

2. Système d'imagerie faciale selon la revendication 1, dans lequel ledit dispositif de commande d'unité de caméra comporte un système de poursuite de face configuré afin de poursuivre ladite image de face.

3. Système d'imagerie faciale selon la revendication 2, dans lequel ledit dispositif de commande d'unité de caméra comporte un logiciel de traitement de données de caméra vidéo configuré pour traiter lesdites images cibles et afin de fournir des données d'image vidéo, et dans lequel ledit système de détection faciale et ledit système de poursuite de face sont configurés pour utiliser lesdites données d'image vidéo pour contribuer à ladite détection et à ladite poursuite de ladite image de face.

4. Système d'imagerie faciale selon l'une quelconque des revendications 1 à 3, dans lequel ladite unité de caméra comporte un système de miroir rotatif destiné à refléter lesdites images cibles et lesdites images de face vers ladite caméra vidéo.

5. Système d'imagerie faciale selon l'une quelconque des revendications 1 à 4, dans lequel ledit système de détection faciale est configuré pour utiliser lesdites données de télémétrie pour contribuer à une détection desdites images de face, lesdites données de télémétrie comprenant une distance et une localisation angulaire.

6. Système d'imagerie faciale selon la revendication 5, dans lequel lesdites données de télémétrie comportent une largeur de ladite cible.

7. Système d'imagerie faciale selon la revendication 2, dans lequel ledit système de poursuite de face est configuré pour utiliser lesdites données de télémétrie pour contribuer à la poursuite desdites images de face, lesdites données de télémétrie comprenant une distance et une localisation angulaire.

8. Système d'imagerie faciale selon la revendication 7, dans lequel lesdites données de télémétrie comportent une largeur de ladite cible.

9. Système d'imagerie faciale selon l'une quelconque des revendications 1 à 8, comportant un système de communication d'unité de caméra destiné à envoyer lesdites images de face capturées vers un dispositif de commande externe à des fins de vérification faciale et/ou reconnaissance faciale.

10. Système d'imagerie faciale selon l'une quelconque des revendications 1 à 9, dans lequel ledit système de détection faciale est configuré pour utiliser la couleur, la forme et la localisation de caractéristiques structurelles dans lesdites images cibles pour identifier ladite image de face.

11. Système d'imagerie faciale selon l'une quelconque des revendications 1 à 10, dans lequel ledit système de détection faciale comporte une détection d'attributs faciaux individuels pour localiser et confirmer des attributs faciaux parmi lesdites images cibles.

12. Système d'imagerie faciale selon l'une quelconque des revendications 1 à 11, dans lequel ledit système de détection faciale comporte un système de détection oculaire qui utilise un illumination infrarouge de ladite cible pour localiser la position des yeux dans lesdites images cibles.

13. Système d'imagerie faciale selon l'une quelconque des revendications 1 à 12, dans lequel ledit système de détection faciale est configuré pour se mettre hors service lorsque ladite image de face est détectée parmi lesdites images cibles.

14. Système d'imagerie faciale selon l'une quelconque des revendications 1 à 13, dans lequel ledit système de capture faciale comporte un système d'évaluation de pose configuré pour évaluer une pose de ladite image de face, afin de déterminer si ladite image de face est de ladite qualité suffisante pour une reconnaissance faciale.

15. Système d'imagerie faciale selon la revendication 14, dans lequel ledit système d'évaluation de pose est capable de déterminer si ladite pose de ladite image de face est une pose frontale.

16. Système d'imagerie faciale selon l'une quelconque des revendications 1 à 15, dans lequel ledit système de capture faciale comportent un système d'évaluation d'éclairage configuré pour évaluer un éclairage de ladite image de face, afin de déterminer si ladite image de face est de ladite qualité suffisante pour une reconnaissance faciale.

17. Système d'imagerie faciale selon la revendication 14, dans lequel ledit système d'évaluation de pose comporte un système d'identification d'attributs faciaux et un système de localisation d'attributs faciaux.

18. Système d'imagerie faciale selon la revendication 16, dans lequel ledit système d'évaluation d'éclairage comporte un système d'analyse de couleur ;

19. Système d'imagerie faciale selon l'une quelconque des revendications 1 à 18, dans lequel ledit système de capture faciale comporte un système d'exposition configuré pour mesurer une exposition de ladite image de face pour commander ledit système de capture faciale afin de déterminer si ladite image de face est de ladite qualité suffisante pour une reconnaissance faciale, ledit système d'exposition étant basé sur une zone de ladite zone d'une taille et d'une localisation ajustables sur ladite image de face.

20. Système d'imagerie faciale selon l'une quelconque des revendications 1 à 19, dans lequel ledit système de détection faciale comporte une détection d'attributs faciaux individuels configuré pour localiser la position des yeux sur lesdites images cibles, et dans lequel ledit système de capture faciale comporte un système d'évaluation de résolution de pixels destiné à déterminer une résolution de pixels minimale de ladite image de face entre lesdits yeux pour déterminer si ladite image de face est de ladite qualité suffisante pour une reconnaissance faciale.

21. Système d'imagerie faciale selon la revendication 1, dans lequel lesdites données de télémétrie sont utilisées pour contribuer à la détection de ladite image de face par ledit système de détection faciale.

22. Système d'imagerie faciale selon la revendication 1, dans lequel lesdites données de télémétrie sont utilisées pour contribuer à la capture de ladite image de face par ledit système de capture faciale.

23. Système d'imagerie faciale selon la revendication 2, dans lequel lesdites données de télémétrie sont utilisées pour contribuer à ladite poursuite de ladite image de face par ledit système de poursuite de face.

24. Système d'imagerie faciale selon la revendication 1, dans lequel ledit dispositif de commande d'unité de caméra comporte un logiciel de traitement de données de caméra vidéo configuré pour traiter lesdites images cibles et pour fournir des données d'images vidéo traitées, et dans lequel ledit système de détection faciale est configuré pour utiliser lesdites données d'images vidéo traitées pour contribuer à ladite détection de ladite image de face.

25. Système d'imagerie faciale selon la revendication 1, dans lequel ledit dispositif de commande d'unité de caméra comporte un logiciel de traitement de données de caméra vidéo configuré pour traiter lesdites images cibles et pour fournir des données d'image vidéo traitées, et dans lequel ledit système de capture faciale est configuré pour utiliser lesdites données d'image vidéo traitées pour contribuer à ladite capture de ladite image de face.

26. Système d'imagerie faciale selon la revendication 2, dans lequel ledit dispositif de commande d'unité de caméra comporte un logiciel de traitement de données de caméra vidéo configuré pour traiter lesdites images cibles et pour fournir des données d'image vidéo traitées et, dans lequel ledit système de poursuite de face est configuré pour utiliser lesdites données d'image vidéo traitées pour contribuer à ladite poursuite de ladite image de face.

27. Système d'imagerie faciale selon l'une quelconque des revendications 24, 25 ou 26, dans lequel lesdites données d'image vidéo traitées sont organisées et mémorisées dans un fichier historique de mouvement.

28. Système d'imagerie faciale selon la revendication 1, dans lequel ladite unité de caméra comporte des attributs automatisés de panoramique horizontal, de zoom et de mouvement basculant vertical.

29. Système d'imagerie faciale selon la revendication 28, dans lequel ledit attribut de panoramique horizontal est mis à disposition par un premier système de miroir rotatif dans une direction horizontale pour refléter lesdites images cibles vers ladite caméra vidéo.

30. Système d'imagerie faciale selon l'une quelconque des revendications 28 ou 29, dans lequel ledit dispositif de commande d'unité de caméra est configuré pour utiliser lesdites données de télémétrie pour contribuer à commander automatiquement les attributs dudit panoramique horizontal, dudit zoom et dudit mouvement basculant vertical.

31. Système d'imagerie faciale selon l'une quelconque des revendications 28 à 30, dans lequel ledit attribut de mouvement basculant vertical est mis à disposition par ledit premier système de miroir, qui est également rotatif dans une direction verticale.

32. Système d'imagerie faciale selon l'une quelconque des revendications 28 à 30, dans lequel ledit attribut de mouvement basculant vertical est mis à disposition par un deuxième système de miroir rotatif dans une direction verticale destiné à refléter lesdites images cibles vers ladite caméra vidéo.

33. Système d'imagerie faciale selon l'une quelconque des revendications 28 à 32, dans lequel ledit dispositif d'unité de caméra comporte un logiciel de traitement de données de caméra vidéo afin de traiter lesdites images cibles et afin de fournir des données d'image vidéo, et dans lequel ledit dispositif de commande d'unité de caméra est configuré pour utiliser lesdites données d'image vidéo afin de contribuer à commander automatiquement les caractéristiques dudit panoramique horizontal, dudit zoom et dudit mouvement basculant vertical.

34. Système d'imagerie faciale selon l'une quelconque des revendications 28 à 33, dans lequel ledit système de détection faciale génère des données d'image de face et ledit dispositif de commande d'unité de caméra est configuré afin d'utiliser lesdites données d'image de face pour contribuer à commander automatiquement les attributs dudit panoramique horizontal, dudit zoom et dudit mouvement basculant vertical.

35. Système d'imagerie faciale selon la revendication 2, dans lequel ladite unité de caméra comporte des attributs automatisés de panoramique horizontal, de zoom et de mouvement basculant vertical, et dans lequel ledit système de poursuite de face génère des données de poursuite de face et ledit dispositif de commande d'unité de caméra est configuré afin d'utiliser lesdites données de poursuite de face pour contribuer à commander automatiquement les attributs dudit panoramique horizontal, dudit zoom et dudit mouvement basculant vertical.
